# EUROPÄISCHE PATENTANMELDUNG

(11) **EP 4 487 821 A1**
(43) Veröffentlichungstag der Anmeldung: **08.01.2025**
(21) Anmeldenummer: 24186072.5
(22) Anmeldetag: 02.07.2024
(51) Int. Cl.: A61F 2/74, A61F 2/50, A61F 2/60, A61F 2/64, A61F 2/68, F16K 1/34, F16K 3/24, F16K 3/314, F16K 3/316, F16K 31/12, F16K 31/14

(54) **STELLVENTIL**

(30) Priorität: 04.07.2023 DE 102023117664
(71) Anmelder: Otto Bock Healthcare Products GmbH, 1110 Wien (AT)
(72) Erfinder: SZTRANCSIK, Zsolt Tamas, 1110 Wien (AT); SCHUH, Andreas, 1110 Wien (AT); BOITEN, Herman, 1110 Wien (AT)
(74) Vertreter: Gramm, Lins & Partner Patent- und Rechtsanwälte PartGmbB

(57) **Zusammenfassung**

Stellventil (10) für einen hydraulischen Aktuatorin einem Strömungskanal (1),
- das Stellventil weist eine Ventilhülse (20) und einen Ventilkörper (30) auf,
- die Ventilhülse weist eine Hülsenwand (22) auf, die einen Aufnahmeraum (23) ausbildet, in der Hülsenwand ist zumindest eine radial ausgerichtete Hülsenwandöffnung (24, 25, 26) ausgebildet,
- der Ventilkörper ist innerhalb des Aufnahmeraumes der Ventilhülse verlagerbar zwischen einer Schließstellung und einer Offenstellung angeordnet und weist einen durch den Ventilkörper gebildeten Hohlraum (31) auf,
- der Ventilkörper weist zumindest eine Ventilkörperwand (32) auf, die der zumindest einen Hülsenwandöffnung gegenüberliegt und in der eine Durchlassöffnung (34, 35, 36) ausgebildet ist, die in der Offenstellung der korrespondierenden Hülsenwandöffnung gegenüberliegt, **wobei** in der Hülsenwand und/oder der Ventilkörperwand zumindest ein Kanal ausgebildet ist und der in der Hülsenwand ausgebildete Kanal (122) an der Hülsenwandöffnung und der an der Ventilkörperwand ausgebildete Kanal (132) an der Durchlassöffnung mündet.

## Beschreibung

Die Erfindung betrifft Stellventil für einen hydraulischen Aktuator, insbesondere für orthopädietechnische Einrichtungen, das in einem Strömungskanal angeordnet und einen Einlass und einen Auslass für ein Hydraulikfluid aufweist. Das Stellventil weist eine Ventilhülse und einen Ventilkörper auf, die Ventilhülse weist eine Hülsenwand auf, die einen Aufnahmeraum ausbildet und in der zumindest eine radial ausgerichtete Hülsenwandöffnung ausgebildet ist, der Ventilkörper ist innerhalb des Aufnahmeraumes der Ventilhülse verlagerbar zwischen einer Schließstellung und einer Offenstellung angeordnet, mündet in einen durch den Ventilkörper gebildeten Hohlraum und weist zumindest eine Ventilkörperwand auf, die der zumindest einen Hülsenwandöffnung gegenüberliegt und in der eine Durchlassöffnung ausgebildet ist, die in der Offenstellung der korrespondierenden Hülsenwandöffnung gegenüberliegt. Der Hohlraum mündet je nach Strömungsrichtung in einen Einlass oder einen Auslass. Strömt das Hydraulikfluid von der Ventilhülse in dem Hohlraum, mündet der Hohlraum in einen Auslass, strömt das Hydraulikfluid umgekehrt in den Hohlraum und durch die Durchlassöffnung und die Hülsenwandöffnung, mündet der Hohlraum in den Einlass.

Orthopädietechnische Einrichtungen sind insbesondere aber nicht ausschließlich Prothesen, Orthesen und Exoskelette. Prothesen ersetzen nicht oder nicht mehr vorhandene Gliedmaßen. Orthesen und Exoskelette werden an Gliedmaßen festgelegt und unterstützen, beeinflussen oder begrenzen bestimmte Bewegungen oder Stellungen von Gliedmaßen zueinander. Bei gelenkübergreifenden Orthesen oder Exoskeletten werden ein Oberteil an einer proximalen Gliedmaße und ein Unterteil an einer distalen Gliedmaße festgelegt und sind über ein Gelenk schwenkbar miteinander verbunden.

Prothesen können ein proximales Oberteil und ein distales Unterteil aufweisen, zwischen denen eine Gelenkeinrichtung angeordnet ist, um eine schwenkbare Verbindung zwischen Oberteil und Unterteil zu erreichen.

Zwischen einem proximalen Oberteil und einem distalen Unterteil einer orthopädietechnischen Einrichtung können Aktuatoren oder Widerstandseinrichtungen angeordnet sein, die eine Verschwenkbewegung zwischen dem Oberteil und dem Unterteil beeinflussen. Die Beeinflussung kann in der Bereitstellung eines Widerstandes stattfinden, beispielsweise über einen Hydraulikdämpfer oder einen Pneumatikdämpfer. Zur aktiven Unterstützung einer Bewegung ist ein Antrieb oder ein Kraftspeicher der orthopädietechnischen Einrichtung zugeordnet, um eine Bewegung aktiv zu bewirken oder um Bewegungsenergie umzuwandeln und zu speichern. Alternativ zu Gelenkeinrichtungen können die orthopädietechnischen Einrichtungen auch eine Linearverlagerung zweier Komponenten zueinander beeinflussen.

Um die Bewegung zwischen zwei Komponenten, beispielsweise einem Oberteil und einem Unterteil einer Gelenkeinrichtung, beeinflussen zu können, sind in einem Strömungskanal des hydraulischen Aktuators, der als hydraulischer Antrieb oder passiver Dämpfer ausgestaltet sein kann, Einrichtungen zur Beeinflussung des Strömungswiderstandes angeordnet. Häufig sind in einem Strömungskanal zwischen zwei Hydraulikkammern, die von einem Kolben getrennt sind, ein oder mehrere Stellventile angeordnet, über die der Strömungsquerschnitt eingestellt werden kann. Die Verstellung kann insbesondere während der Benutzung der orthopädietechnischen Einrichtung sensorbasiert stattfinden. Dazu werden Sensordaten erfasst und an eine Steuerungseinrichtung weitergeleitet. In der Steuerungseinrichtung werden die Sensordaten ausgewertet und während der Benutzung der orthopädietechnischen Einrichtung in Stellsignale umgewandelt, aufgrund derer das Stellventil verstellt wird. Damit ist es möglich, die orthopädietechnische Einrichtung an die jeweilige Nutzungssituation anzupassen.

Aufgrund der vergleichsweise geringen Abmessungen innerhalb orthopädietechnischer Einrichtungen und der dort herrschenden hohen Drücke werden innerhalb der Strömungskanäle und damit auch innerhalb der Stellventile hohe Strömungsgeschwindigkeiten erreicht. Dies führt zu mitunter hohen Geräuschbelastungen, die sowohl für den Nutzer der orthopädietechnischen Einrichtung als auch für die Umgebung wahrnehmbar sind. Darüber hinaus sind hydraulische Aktuatoren in den Bereichen der Stellventile anfällig für Strömungsverluste, was zu einem erhöhten Grundwiderstand innerhalb der orthopädietechnischen Einrichtung führt. Dadurch verschlechtert sich die Präzision der Steuerung und das Verschwenkverhalten der Gelenkkomponenten bzw. das Bewegungsverhalten der Komponenten, zwischen denen der hydraulische Aktuator angeordnet ist, wird permanent beeinflusst. Die bei kleinen Abmessungen der Strömungskanäle und hohen Drücken auftretenden Strömungsgeschwindigkeiten führen mitunter zu turbulenten Strömungen und ungewollten, lokalen Temperaturspitzen und zu einer ungünstigen Verteilung der Wärme in der orthopädietechnischen Einrichtung. Temperaturspitzen führen zu einer ungewollten Veränderung der Viskosität des Hydraulikfluides.

Aufgabe der vorliegenden Erfindung ist es ein Stellventil bereitzustellen, mit dem eine komfortablere Nutzung und ein verbessertes Steuerungsverhalten bereitgestellt werden kann.

Diese Aufgabe wird mit einem Stellventil mit den Merkmalen des nebengeordneten Anspruchs gelöst. Vorteilhafte Ausgestaltungen und Weiterbildungen der Erfindung sind in den Unteransprüchen, der Beschreibung sowie den Figuren offenbart.

Das Stellventil für einen hydraulischen Aktuator, insbesondere für orthopädietechnische Einrichtungen, das in einem Strömungskanal angeordnet und einen Einlass und einen Auslass für ein Hydraulikfluid aufweist, mit einer Ventilhülse und einen Ventilkörper, wobei die Ventilhülse eine Hülsenwand aufweist, die einen Aufnahmeraum ausbildet und in der zumindest eine radial ausgerichtete Hülsenwandöffnung ausgebildet ist und der Ventilkörper innerhalb des Aufnahmeraumes der Ventilhülse verlagerbar zwischen einer Schließstellung und einer Offenstellung angeordnet ist und in einen durch den Ventilkörper gebildeten Hohlraum mündet und zumindest eine Ventilkörperwand aufweist, die der zumindest einen Hülsenwandöffnung gegenüberliegt und in der eine Durchlassöffnung ausgebildet ist, die in der Offenstellung der korrespondierenden Hülsenwandöffnung gegenüberliegt zeichnet sich dadurch aus, dass in der Hülsenwand und/oder der Ventilkörperwand zumindest ein Kanal ausgebildet ist und der in der Hülsenwand ausgebildete Kanal an der Hülsenwandöffnung und der an der Ventilkörperwand ausgebildete Kanal an der Durchlassöffnung mündet.

Bei dem Stellventil strömt Hydraulikfluid z.B. radial von außen durch die zunächst eine Hülsenwandöffnung und damit Einlassöffnung in der Ventilhülse in Richtung auf den Ventilkörper und bei einer entsprechenden Stellung des Ventilkörpers relativ zu der Ventilhülse durch die Durchlassöffnung. Eine sehr präzise und eindeutig beeinflussbare Strömung lässt sich einfacher bei laminaren Strömungen erreichen. Stehen sich die Hülsenwandöffnung und die Durchlassöffnung direkt gegenüber, hat das Stellventil den maximalen Durchfluss. Wird der Ventilkörper gegenüber der Ventilhülse verlagert, beispielsweise verdreht oder verschoben, wird der Strömungsquerschnitt verringert. Aufgrund des zumindest einen Kanals, der in der Hülsenwand oder Ventilkörperwand an der Außenseite angeordnet oder ausgebildet ist, wird das Fluid über eine Wegstrecke zu der Durchlassöffnung oder Hülsenwandöffnung geführt, wobei die Strömungscharakteristik dieses Weges laminar ist. Dadurch ist es bei einem verringerten Strömungsquerschnitt aufgrund der eingestellten Verdrehung oder Verlagerung des Ventilkörpers relativ zu der Ventilhülse möglich, eine starke lokale Erhitzung in dem Bereich der Drosselstelle aufgrund turbulenter Strömungen zu verhindern. Darüber hinaus werden durch die laminare Strömungen keine oder nur geringere Geräusche im Vergleich zu turbulenten Strömungen hervorgerufen, was die Nutzung der orthopädietechnischen Einrichtung für den Nutzer angenehmer macht. Es können mehrere Kanäle in der Hülsenwand und/oder der Ventilkörperwand ausgebildet sein und in Richtung auf die jeweilige Öffnung innerhalb der Wand verlaufen. Diese können geradlinig oder insbesondere gekrümmt ausgebildet sein. Der Kanalquerschnitt ist insbesondere so ausgebildet, dass eine laminare Strömung vorhanden ist. Dementsprechend ist eine geradlinige Führung des Kanals an der jeweiligen Wand vorteilhaft, wobei bei einer gekrümmten Wand der Strömungskanal naturgemäß um die Längserstreckung herum gekrümmt ist.

In einer Ausgestaltung liegt ein an der Hülsenwand ausgebildeter Kanal einem an der Ventilkörperwand ausgebildeten Kanal gegenüber, sodass sich die beiden Kanäle ergänzen. Die Kanäle können so angeordnet sein, dass sie auf dem gesamten Verstellweg des Ventilkörpers relativ zu der Ventilhülse einander gegenüberliegen. Aufgrund der begrenzten Länge der Kanäle findet keine vollständige Überdeckung und Ergänzung der Kanäle die gesamte Länge statt. Alternativ ist auch eine Überdeckung nur über einen Teilbereich der Länge des Kanals möglich und vorgesehen.

In einer Ausgestaltung ist der Kanal zumindest teilweise als nach außen offener Kanal oder Rinne ausgebildet, sodass sich beispielsweise bei einem halbkreisförmigen Querschnitt ein gegenüberliegender Kanal diesen zu einem geschlossenen Kanal mit einem kreisförmigen Querschnitt ergänzt. Bei einer glatten gegenüberliegenden Wand wird der Kanal oder die Rinne durch die gegenüberliegende Wand geschlossen. Bei einem Kanal in der Hülsenwand wird dieser dann durch die Ventilkörperwand geschlossen und umgekehrt.

In einer Ausgestaltung vergrößert sich der Kanal in Richtung auf die Durchlassöffnung bzw. Hülsenwandöffnung und bewirkt eine Verringerung der Strömungsgeschwindigkeit in Richtung auf diejenige Öffnung, die das Hydraulikfluid zu einem Auslass leitet.

In einer Ausgestaltung ist der Hohlraum bis auf die zumindest eine Durchlassöffnung und eine Zugangsöffnung geschlossen ausgebildet, wobei insbesondere die der Zugangsöffnung gegenüberliegende Stirnwand des Hohlraumes geschlossen ist. Innerhalb der Ventilkörperwand können mehrere Durchlassöffnungen korrespondierend zu Hülsenwandöffnungen in der Hülsenwand ausgebildet sein, um mehrere Teilströme des Hydraulikfluides in den Hohlraum einleiten oder daraus herausleiten zu können. Die ansonsten geschlossene Ausgestaltung des Hohlraumes fördert die effektive Umleitung und Beeinflussung der Strömungsgeschwindigkeiten des Hydraulikfluides.

In einer Ausgestaltung ist der Ventilkörper drehbar oder verschieblich in der Ventilhülse gelagert. Durch das Verdrehen oder das Verschieben des Ventilkörpers in der Ventilhülse werden unterschiedliche Querschnitte für das Hydraulikfluid in dem Hohlraum des Ventilkörpers bereitgestellt. Der insgesamt vorhandene Einlassquerschnitt ergibt sich aus den sich überschneidenden Geometrien in der Hülsenwandöffnung mit der jeweils gegenüberliegenden Durchlassöffnung. Bei einer statischen Ventilhülse und einem dazu relativ beweglich gelagerten Ventilkörper kann durch die Relativbewegung der Einlassquerschnitt vergrößert oder verringert und im Extremfall vollständig geschlossen werden. Über eine entsprechende Geometrie sowohl der Hülsenwandöffnung als auch der Durchlassöffnung ist es möglich, für jeden Verdrehwinkel bzw. für jede Verschiebeposition einen optimalen Querschnitt für die Einströmöffnung zu erzeugen. Beispielsweise kann es ausgehend von einer Schließstellung sinnvoll und wünschenswert sein, zunächst nur eine langsame Vergrößerung des Einlassquerschnittes zu bewirken und erst ab einem vorbestimmten Verdrehwinkel oder ab einer Verschiebeposition die Vergrößerung progressiver ansteigen zu lassen, um dann den Anstieg der Vergrößerung bis zur vollständigen Offenstellung zu verringern, um eine Feinabstimmung zu ermöglichen. Umgekehrt kann auch in einem mittleren Öffnungsbereich die Veränderung pro Einheit des Verschiebeweges oder pro Verstellwinkel klein gewählt werden, um eine Feineinstellung in einem mittleren Dämpfungsbereich oder Aktivierungsbereich zu ermöglichen.

In einer Ausgestaltung ist der Ventilkörper mit einer Verstelleinrichtung gekoppelt, mit der es möglich ist, den Ventilkörper in die jeweils gewünschte Position einzustellen bzw. zu bewegen und in der jeweils gewünschten Position zu halten. Die Verstelleinrichtung kann mit einem manuellen oder motorischen Antrieb gekoppelt sein, auch mit einem mechanischen Antrieb kann die Verstelleinrichtung gekoppelt sein, beispielsweise um ein wegeabhängiges oder belastungsabhängiges Öffnen oder Schließen des Einlassquerschnittes zu bewirken.

Die Ventilhülse kann als Teil eines Gehäuses des hydraulischen Aktuators, beispielsweise als Teil eines Dämpfergehäuses, ausgebildet sein. Alternativ ist die Ventilhülse als ein separates Bauteil ausgebildet, sodass ein Stellventil aus Ventilhülse, Ventilkörper und Diffusoreinsatz als modulare Einheit ausgebildet und separat gefertigt werden kann. Ein solches Stellventil kann dann in den hydraulischen Aktuator eingesetzt und gegebenenfalls mit der Verstelleinrichtung gekoppelt werden.

In einer Ausgestaltung ist die Hülsenwandöffnung und/oder die Durchlassöffnung als Schlitz oder Bohrung ausgebildet, wobei mehrere Öffnungen in den jeweiligen Wänden angeordnet oder ausgebildet sein können, um das Hindurchtreten eines Hydraulikfluides zu ermöglichen. Beispielsweise können mehrere entsprechend angeordnete und ausgerichtete Bohrungen einem Schlitz gegenüberliegen, sodass Hydraulikfluid aus zwei Bohrungen in einen Schlitz eintreten und umgekehrt. Beispielsweise ist der Schlitz in dem Ventilkörper ausgebildet, während die Bohrungen nebeneinander in der Hülsenwand ausgebildet sind.

In einer Ausgestaltung sind die Hülsenwandöffnung und/oder die Durchlassöffnung als Schlitz ausgebildet und schräg zur Längserstreckung des Stellventils orientiert. Der Schlitz kann gradlinig, also mit einer konstanten Steigung, an dem Umfang der Ventilhülse und/oder dem Ventilkörper ausgebildet sein. Alternativ ist der Schlitz gekrümmt oder zumindest bereichsweise gekrümmt, wodurch sich unterschiedliche Überschneidungen von Hülsenwandöffnung und Durchlassöffnung bei gleichmäßiger Verstellbewegung erzielen lassen. Die Breite des Schlitzes kann sich entlang seiner Längserstreckung ändern, um eine Vergrößerung bzw. Verringerung der Breite entlang seiner Längserstreckung oder entlang der Axialerstreckung des Stellventils zu erreichen. Dadurch kann eine angepasste Veränderung des Einlassquerschnittes in den Hohlraum erreicht werden.

In einer Ausgestaltung sind die Kanäle so angeordnet oder ausgebildet, dass in der Schließstellung keine strömungstechnische Verbindung zwischen der Hülsenwandöffnung und der Durchlassöffnung besteht. In der Schließstellung kann auch durch die Kanäle keine Hydraulikflüssigkeit hindurchtreten. Dies wird beispielsweise dadurch erreicht, dass bei einer verdrehbaren Lagerung des Ventilkörpers in der Ventilhülse die Länge des Kanals so ausgebildet ist, dass er vor der Durchlassöffnung bzw. Hülsenwandöffnung endet, wenn sich das Stellventil in der Schließstellung befindet. Sind beispielsweise nur eine Hülsenwandöffnung und eine Durchlassöffnung über den Umfang des Stellventils bei einer verdrehbaren Ausgestaltung angeordnet, beträgt die maximale Umfangslänge des Kanals den Umfang der Wand, in dem der Kanal ausgebildet ist, abzüglich der Breite der Hülsenwandöffnung und der Breite der Durchlassöffnung. Bei einer verschieblichen Lagerung entlang der Längserstreckung beträgt die Länge des Kanals die maximale Verschiebelänge abzüglich der Abmessung der gegenüberliegenden Öffnung. Die Unterbrechung der strömungstechnischen Verbindung kann auch durch eine entsprechende Krümmung und einen entsprechenden Verlauf des Kanals auf der Oberfläche der Hülsenwand oder Ventilkörperwand erfolgen. Die Kanäle überdecken sich in der Schließstellung nicht, eine absolute Abdichtung ist aber aufgrund der üblichen Fertigungstoleranzen und des Ringspaltes zwischen dem Ventilkörper und der Ventilhülse nicht möglich. In der Anwendung findet aber nahezu kein nennenswerter Volumenstrom in der Schließstellung statt.

In einer Ausgestaltung ist mündet der Hohlraum in einen Auslass und in dem Hohlraum ist ein Diffusoreinsatz angeordnet, der das Hydraulikfluid von der Durchlassöffnung in Richtung auf den Auslass umgelenkt. Durch den Diffusoreinsatz wird verhindert, dass das radial einströmende Hydraulikfluid auf die gegenüberliegende Wand des Ventilkörpers auftrifft oder sich mit einem eindringenden Hydraulikstrom aus einer weiteren Hülsenwandöffnung mischt bzw. auf diesen auftrifft. Dies würde zu turbulenten Strömungen innerhalb des Hohlraumes innerhalb des Ventilkörpers und damit zu hohen Geräuschbelastungen sowie einem erhöhten Strömungswiderstand, insbesondere bei vollständig geöffneten Durchlassöffnungen führen. Der Diffusoreinsatz innerhalb des Hohlraumes des Ventilkörpers lenkt das Hydraulikfluid um, sodass es statt rechtwinklig auf eine Ventilkörperwand aufzutreffen in Richtung auf den Auslass umgelenkt wird. Dadurch wird die Bildung von Toträumen und Verwirbelungen innerhalb des Hohlraumes des Ventilkörpers vermieden, sodass der Strömungswiderstand und die Geräuschentwicklung verringert werden.

In einer Ausgestaltung ist der Diffusoreinsatz separat von dem Ventilkörper gefertigt und an oder in dem Ventilkörper befestigt. Dadurch ist es möglich, den Diffusoreinsatz aus einem anderen Material als dem Ventilkörper herzustellen, insbesondere aus einem anderen Material als die Ventilkörperwand, in der die Hülsenwandöffnung oder die Hülsenwandöffnungen ausgebildet ist bzw. sind. Die separate Herstellung des Diffusoreinsatzes ermöglicht zudem eine Anpassung der äußeren Kontur bzw. der Geometrie des Diffusoreinsatzes an unterschiedliche Einsatzbedingungen oder Modelle sowie unterschiedliche hydraulischer Aktuatoren. Darüber hinaus ist das Fertigen komplexer dreidimensionaler Strukturen mit einer freien Zugänglichkeit von außen leichter als innerhalb eines hülsenartigen Ventilkörpers. Alternativ ist der der Diffusoreinsatz integraler Bestandteil des Ventilkörpers und beispielsweise im Rahmen eines additiven Fertigungsverfahrens hergestellt, bei dem durch einen schichtweisen Aufbau von Materiallagen komplexe dreidimensionale Strukturen während des Urformverfahrens hergestellt werden können.

In einer Ausgestaltung ist der Diffusoreinsatz in Richtung auf den Auslass sich verjüngend ausgebildet, um langsam eine Vergrößerung des Strömungsquerschnittes in Richtung auf den Auslass zu ermöglichen.

Der Ventilkörper weist in einer Ausgestaltung mehrere Durchlassöffnungen auf, der Diffusoreinsatz weist Trennwände auf, die zwischen den Durchlassöffnungen angeordnet sind. Dadurch ist es möglich, die Strömungskanäle der jeweiligen Durchlassöffnungen zu trennen und einzeln umzuleiten. Innerhalb von Strömungskanälen können unterschiedliche Strömungsbedingungen herrschen, sodass auch das Umlenken der Teilströme auf unterschiedliche Arten und Weisen erfolgen kann, um eine optimale Beeinflussung und Umlenkung der Teilströme zu erreichen.

In einer Ausgestaltung erstrecken sich die Trennwände nicht bis zu dem Ende des Hohlraumen oder dem Auslass, sondern unterteilen den Hohlraum des Ventilkörpers nur über eine Teillänge in Axialerstreckung, sodass noch innerhalb des Hohlraumes die einzelnen Teilströme des Hydraulikfluides zusammengeführt werden. Dabei ist es vorteilhaft, wenn sich die Trennwände in Richtung auf den Auslass verjüngen, sodass sich eine allmähliche Aufweitung des Querschnittes für den Fluidstrom ergibt, um eine möglichst wenig turbulente Strömung beizubehalten oder zu erzeugen, insbesondere um eine laminare Strömung beizubehalten oder zu erzeugen. In einer Alternative erstrecken sich die Trennwände bis zu dem Auslass, so dass die einzelnen Teilströme sich erst nach dem Auslass vereinigen.

Ein Einlass zu dem Stellventil und ein Auslass aus dem Stellventil können in strömungstechnischer Verbindung mit einer Kolben-Zylinder-Einheit der orthopädietechnischen Einrichtung stehen und mit zwei durch den Kolben der Kolben-Zylinder-Einheit getrennten Kammern verbunden sein. Dadurch wird ein hydraulischer Kreislauf zwischen dem Einlass und dem Auslass hergestellt. Grundsätzlich ist es möglich, dass nur ein Strömungskanal zwischen den Kammern der Kolben-Zylinder-Einheit vorhanden ist, in der das Stellventil angeordnet ist. Dann ist das Stellventil ggf. mit dem Diffusoreinsatz nur in einer Richtung wirksam. In der entgegengesetzten Richtung wäre dann der Einlass der Auslass und das Hydraulikfluid würde mit einem hohen Druck durch den Hohlraum und die Durchlassöffnungen zurück in die Hülsenwandöffnung strömen. Bevorzugt sind mehrere Stellventile in den hydraulischen Verbindungen zwischen den Kammern angeordnet, insbesondere um eine getrennte Beeinflussung der Verstellbewegungen zu ermöglichen.

Der Kanal oder die Rinne bzw. die Rinnen, sind in einer Ausgestaltung derart ausgeführt ist, dass sich eine laminare Strömung in dem Kanal ausbildet und ein proportionaler Zusammenhang zwischen Druck und Volumenstrom vorliegt. Dies wird insbesondere dadurch erreicht, dass der Kanal eine konstante Breite und/oder eine konstante Tiefe aufweist.

Die Erfindung betrifft ebenfalls einen hydraulischen Aktuator mit einem oben beschriebenen Stellventil.

Nachfolgend werden Ausführungsbeispiele der Erfindung anhand der beigefügten Figuren näher erläutert. Es zeigen:
Figur 1 - eine schematische Darstellung einer angelegten Prothese;
Figur 2 - eine schematische Darstellung eines Prothesenbeines;
Figur 3 - eine Schnittdarstellung eines Stellventils;
Figur 4 - eine Einzeldarstellung eines Diffusoreinsatzes;
Figur 5 - eine Schnittdarstellung einer Stellventilanordnung;
Figur 6 - eine perspektivische Darstellung eines Diffusoreinsatzes;
Figur 7 - eine Ansicht des Diffusoreinsatzes von schräg hinten;
Figur 8 - eine Schnittdarstellung durch einen Ventilkörper mit Diffusoreinsatz;
Figur 9 - eine Explosionsdarstellung eines Stellventils;
Figur 10 - das Stellventil gemäß Figur 9 in geöffneter Stellung;
Figur 11 - eine perspektivische Darstellung des Stellventils gemäß Figur 10;
Figur 12 - eine Längsschnittdarstellung durch ein Stellventil;
Figur 13 - eine Querschnittsdarstellung durch ein geöffnetes Stellventil;
Figur 14- eine perspektivische Darstellung eines geschlossenen Stellventils;
Figur 15 - Schnittdarstellungen durch das Stellventil gemäß Figur 14;
Figur 16 - ein Stellventil in perspektivischer Darstellung;
Figur 17 - der Stellventil gemäß Figur 16 in teiltransparenter Darstellung;
Figur 18 - das Stellventil gemäß Figur 17 in teilweise geschlossener Darstellung;
Figur 19 - eine Darstellung des Strömungsverlaufes in dem Stellventil gemäß Figur18;
Figur 20 - das Stellventil gemäß Figur 16 in Schnittdarstellung;
Figur 21 - das Stellventil mit Kanal; sowie
Figur 22 - ein Ventilkörper in Einzeldarstellung.

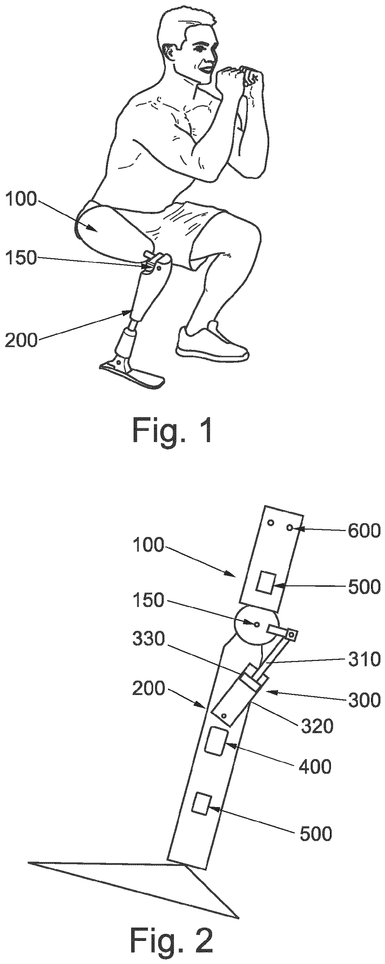

In der Figur 1 ist eine nutzende Person einer orthopädietechnischen Gelenkeinrichtung einer unteren Extremität mit einem Oberteil 100, das einen Prothesenschaft aufweist, und einem Unterteil 200 in Gestalt eines Unterschenkelteils mit einem distal daran befestigten Prothesenfuß gezeigt. Das Oberteil 100 ist relativ zu dem Unterteil 200 um eine Schwenkachse 150 verschwenkbar.

In der Figur 2 ist in einer schematischen Darstellung eine orthopädietechnische Gelenkeinrichtung in Gestalt einer Prothese der unteren Extremität dargestellt. Die orthopädietechnische Gelenkeinrichtung weist ein Oberteil 100 und ein Unterteil 200 auf, die schwenkbar um eine Schwenkachse 150 aneinander gelagert sind. Zur Beeinflussung der relativen Verschwenkung des Oberteils 100 zu dem Unterteil 200 um die Schwenkachse 150 ist an dem Oberteil 100 und an dem Unterteil 200 ein Aktuator 300 angeordnet, der in dem dargestellten Ausführungsbeispiel als ein Hydraulikdämpfer ausgestaltet ist. Der Hydraulikdämpfer 300 ist als eine Kolben-Zylinder-Einheit ausgebildet und weist in einem Gehäuse einen Zylinder auf, der durch einen Kolben 330, der mit einer Kolbenstange gekoppelt ist, in zwei Hydraulikkammern 310, 320 unterteilt wird. Zwischen den beiden Hydraulikkammern 310, 320 ist zumindest eine strömungstechnische Verbindung ausgebildet, damit Hydraulikfluid bei der Vergrößerung der einen Kammer in diese hineinströmen und umgekehrt bei einer Verringerung der Kammer aus dieser herausströmen und in die gegenüberliegende Kammer hineinströmen kann. In der nicht dargestellten strömungstechnischen Verbindung zwischen den beiden Kammern 310, 320 ist ein Stellventil angeordnet, das weiter unten näher beschrieben wird. In alternativen Ausgestaltungen kann der Aktuator 300 als ein aktiver Aktuator mit einem Antrieb, beispielsweise einem Elektromotor, einem Kraftspeicher oder einer anderen Art und Weise des Antriebes ausgestattet sein, um eine Verschwenkbewegung des Oberteils 100 relativ zu dem Unterteil 200 zu beeinflussen. In dem dargestellten Ausführungsbeispiel ist der Aktuator 300 als passiver Aktuator ausgebildet und stellt einen Widerstand gegen eine Flexionsbewegung und gegebenenfalls einer Extensionsbewegung zur Verfügung. Der Aktuator 300 ist mit einer Steuerungseinrichtung 400 gekoppelt, die in dem dargestellten Ausführungsbeispiel an dem Unterteil 200 angeordnet ist. Die Steuerungseinrichtung 400 ist mit Sensoren 500 gekoppelt, die an dem Oberteil 100 und/oder dem Unterteil 200 angeordnet sind. Die Sensoren 500 erfassen Zustandsgrößen der Gelenkeinrichtung, z.B. Positionen, Lagen, Kräfte, Momente, Beschleunigungen oder Stellungen von Komponenten im Raum oder zueinander, wobei eine Vielzahl von Sensoren eingesetzt werden kann, um die gewünschten Zustandsgrößen der orthopädietechnischen Gelenkeinrichtung zu erfassen. Die Sensoren 500 sind mit der Steuerungseinrichtung 400 gekoppelt und übermitteln entsprechende Sensordaten oder Sensorsignale an die Steuerungseinrichtung 400, wobei auf Grundlage der Sensorwerte oder Sensordaten der Aktuator 300 hinsichtlich seiner Fähigkeit, die Verschwenkbewegung oder Verschwenkbarkeit des Oberteils 100 relativ zu dem Unterteil 200 zu beeinflussen, verändert wird. Beispielsweise wird der Flexionswiderstand und/oder Extensionswiderstand in Abhängigkeit von den Sensorwerten verändert. Dazu werden Ventile oder Drosseln verstellt. In alternativen Ausführungsformen können Magnetfelder verändert werden, um magnetorheologische Fluide hinsichtlich ihrer Viskosität zu verändern. Bei einer mechanischen Bremseinrichtung können Bremskräfte erhöht oder verringert werden, um ein angepasstes Widerstandsverhalten des Aktuators 300 zu erzeugen. Bei einem aktiven Antrieb, der zumindest einen elektromechanischen Aktuator aufweist, können über Ströme und Spannungen Widerstände gegen eine Bewegung aufgebracht oder aber auch Bewegungen unterstützt werden. Neben dem Aufbringen von Momenten und Momentenverläufen können über Steuerungsalgorithmen und Sensorinformationen über die Verschwenkbewegung von Oberteil 100 und Unterteil 200 Trajektorien verfolgt oder Systemeigenschaften im Sinne einer Impedanz- oder Admittanz-Regelung emuliert werden. Zum Beispiel kann das Verhalten einer linearen oder nichtlinearen Feder, das Verhalten eines Dämpfers oder eine Trägheit emuliert werden oder eine Kombination mehrerer Eigenschaften, wodurch eine Verschwenkbewegung entgegen einer Flexion beeinflusst werden kann. Eine solche Ansteuerung bietet ein hohes Maß an Flexibilität. Durch derartige Steuerungen können Verschwenkbewegungen auch aktiv unterstützt werden. Wird über einen Aktuator ein Moment oder eine Kraft aufgebracht, ohne dass eine Verschwenkung stattfindet, bspw. weil externe und interne Kräfte im Gleichgewicht stehen, wird der Verschwenkbewegung ebenfalls ein Widerstand entgegengebracht. Über Aktuatoren können auch Energiespeicher aktiviert und deaktiviert werden, zum Beispiel ein hydraulischer Federspeicher, Übersetzungen von Antrieben verändert und/oder diese ein- oder ausgekoppelt werden. Diese Arten der Aktuierung können ebenfalls zur Beeinflussung der Verschwenkbewegung angewendet werden. Widerstände sind Kräfte und Momente, die durch Aktuatoren aufgebracht werden, um eine Verschwenkbewegung zu beeinflussen. Ein Widerstand kann einer Verschwenkbewegung entgegengesetzt sein, aber auch eine Unterstützung einer Verschwenkbewegung sein, das heißt, in Richtung der Verschwenkung wirken.

An dem Oberteil 100 ausgebildet oder daran befestigt, beispielsweise an einem Prothesenschaft oder an einer Halterung, sind Biosignalsensoren 600, die zur Erfassung von Muskelaktivitäten oder einer Ansteuerung eines Muskels oder der Muskulatur ausgebildet sind und Biosignale erzeugen, die an die Steuerungseinrichtung 400 übermittelt werden. Die Biosignalsensoren 600, die als Ableiterelektroden ausgebildet sein können, sind mit der Steuerungseinrichtung 400 über eine leitende Verbindung oder drahtlos gekoppelt und übermitteln entsprechende Signale an die Steuerungseinrichtung 400, die den Aktuator 300 ebenfalls auf der Grundlage der Biosignale aktiviert, deaktiviert oder moduliert, um den Widerstand zu verändern oder das Bewegungsverhalten oder die Verlagerbarkeit der Komponenten zueinander zu beeinflussen.

Abweichend von der Ausgestaltung der orthopädietechnischen Gelenkeinrichtung als Prothese kann diese auch als Orthese ausgebildet sein, wobei statt des Unterschenkelteils als Unterteil 200 eine Unterschenkelschiene an einem künstlichen Kniegelenk ausgebildet ist. Das Oberteil 100 ist dann eine Oberschenkelschiene, die über entsprechende Befestigungseinrichtungen wie Gurte, Schalen oder Manschetten an dem Oberschenkel festgelegt werden kann. Die Biosignalsensoren 600 sind entweder separat an dem Oberschenkel oder einer anderen Muskulaturgruppe befestigt und können auch an der Befestigungseinrichtung angeordnet sein. Die orthopädietechnische Gelenkeinrichtung kann auch als Exoskelett als Sonderform einer Orthese ausgebildet sein. Alternativ zu der dargestellten Ausführungsform als orthopädietechnische Gelenkeinrichtung der unteren Extremität kann die orthopädietechnische Gelenkeinrichtung auch für eine obere Extremität ausgebildet sein, beispielsweise als künstliches Schultergelenk oder künstliches Ellenbogengelenk für einen Prothesenarm oder als Orthese bzw. ein Exoskelett für einen Arm.

Die Aktivierung, Deaktivierung und gegebenenfalls das Modulieren des Aktuators zur Beeinflussung einer Verschwenkbewegung oder der Verschwenkbarkeit der Gelenkeinrichtung erfolgt auf der Grundlage zumindest eines Signals von einem Sensor dergestalt, dass die Beeinflussung der Verschwenkbewegung oder Verschwenkbarkeit gegen eine Flexion erhöht wird, wenn ein entsprechendes Sensorsignal vorliegt. Unter einer Beeinflussung der Verschwenkbewegung wird auch verstanden, dass eine Verschwenkbewegung gesperrt wird, sodass das Unterteil 200 relativ zu dem Oberteil 100 verriegelt wird und keine Relativbewegung zwischen den beiden Teilen stattfindet und stattfinden kann. Die Beeinflussung der Verschwenkbarkeit des Oberteils 100 relativ zu dem Unterteil 200 kann also in einer Sperrung des Gelenks in Flexionsrichtung oder einer Erhöhung des Flexionswiderstandes erfolgen. Die Beeinflussung wird entweder durch einen passiven oder einen aktiven Aktuator durchgeführt. Um den erhöhten Flexionswiderstand der Gelenkeinrichtung wieder zu verringern, erfolgt dies auf der Grundlage von Sensorwerten, also von Werten, die von dem Sensor 500 oder den Sensoren 500 und/oder Biosignalsensoren 600 an die Steuerungseinrichtung 400 übermittelt werden. Eine solche Steuerung der orthopädietechnischen Gelenkeinrichtung, beispielsweise einer unteren Extremität ist beispielsweise für eine Stehfunktion vorteilhaft und sinnvoll, bei der eine Flexion, beispielsweise des Kniegelenkes, erschwert oder gesperrt werden soll. Die Beeinflussung der Verschwenkbarkeit erfolgt beispielsweise über eine Verstellung eines Stellventils.

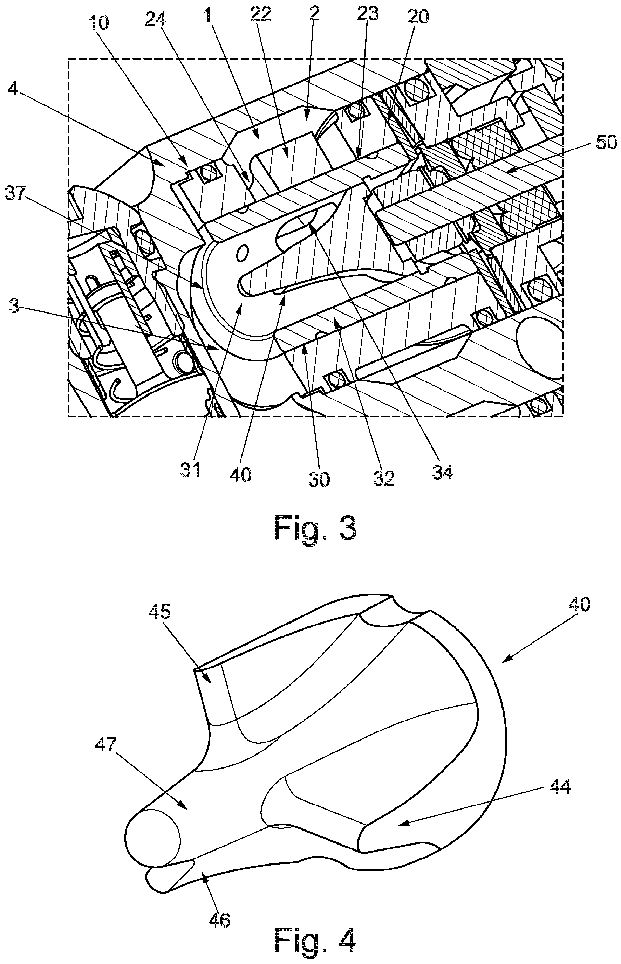

In der Figur 3 ist in einer Schnittdarstellung ein Stellventil 10 dargestellt, das in einem Strömungskanal 1 angeordnet ist, der sich zwischen einer der beiden Kammern 310, 320 befindet, die nicht dargestellt sind. Innerhalb des Strömungskanals wird Hydraulikfluid zu einem Einlass 2 des Stellventils 10 transportiert. Sobald das Hydraulikfluid durch das Stellventil 10 durchgeströmt ist, verlässt es dieses durch einen Auslass 3. Das Stellventil 10 weist eine Ventilhülse 20 auf, die in dem dargestellten Ausführungsbeispiel im Wesentlichen zylindrisch ausgebildet ist. Die Ventilhülse 20 weist eine Hülsenwand 22 auf, die einen Aufnahmeraum 23 umgibt. Innerhalb des Aufnahmeraumes 23 ist ein Ventilkörper 30 angeordnet. Hydraulikfluid wird von dem Einlass 2 aus dem Strömungskanal 1 durch Hülsenwandöffnungen 24 in Richtung auf den Ventilkörper 30 geleitet. In der Schnittdarstellung der Figur 3 ist nur eine Hülsenwandöffnung 24 dargestellt, die sich in zwei Teileinlassöffnungen aufteilt, wenn man annimmt, dass das Hydraulikfluid von außen nach innen strömt, umgekehrt wären es Teilauslassöffnungen. Die Hülsenwandöffnung 24 ist radial ausgerichtet, sodass Hydraulikfluid, das von außen durch den Strömungskanal 1, der die Ventilhülse 20 umfänglich umgibt, zu der Hülsenwandöffnung 24 transportiert wird, in das Innere der Ventilhülse 20 gelangen kann. Der Strömungskanal 1 ist ringförmig um die Ventilhülse 20 herum angeordnet, sodass durch mehrere Hülsenwandöffnungen das Hydraulikfluid in Richtung auf den Ventilkörper 30 geleitet werden kann.

Der Ventilkörper 30 ist innerhalb des Aufnahmeraumes 23 der Ventilhülse 20 verlagerbar, in dem dargestellten Ausführungsbeispiel verdrehbar gelagert. Durch die Verdrehung des Ventilkörpers 30 innerhalb der Ventilhülse 20 ist es möglich, das Stellventil 10 zu öffnen und zu schließen. Dazu wird der Ventilkörper 30 zwischen einer Schließstellung und einer Offenstellung verdreht. In den Zwischenstellungen ist das Stellventil 10 teilweise geöffnet bzw. teilweise geschlossen, sodass sich ein verminderter Volumenstrom des Hydraulikfluides einstellt. Damit einhergehend ist ein erhöhter Strömungswiderstand in dem hydraulischen System vorhanden, sodass der Widerstand des hydraulischen Aktuators verstellt werden kann. Um das Hydraulikfluid von dem Einlass 2 zu dem Auslass 3 durch die Hülsenwandöffnung 24 durchtreten zu lassen, weist der Ventilkörper 30 zumindest eine Ventilkörperwand 32 auf, die der Hülsenwandöffnung 24 oder den Hülsenwandöffnungen gegenüberliegt und in der zumindest eine Durchlassöffnung 34 ausgebildet ist, die in einer Offenstellung der korrespondierenden oder gegenüberliegenden Hülsenwandöffnung 24 gegenüberliegt. In der Offenstellung stellt sich aufgrund der Überlagerung der Hülsenwandöffnung 24 und der Durchlassöffnung 34 ein maximaler Querschnitt für die Durchtrittsöffnung für das Hydraulikfluid ein. In der Schließstellung ist der Querschnitt der Durchtrittsöffnung 0, sodass kein Hydraulikfluid von dem Einlass 2 zu dem Auslass 3 hindurchtreten kann. Durch eine Verdrehung des Ventilkörpers 30 innerhalb des Aufnahmeraumes 23 werden unterschiedliche Durchtrittsquerschnitte erzeugt, um den gewünschten hydraulischen Widerstand einzustellen.

Alternativ zu einer Verdrehung, die in der Figur 3 dargestellt ist, kann die Veränderung des Durchtrittsquerschnittes auch durch eine Verschiebung des Ventilkörpers 30 relativ zu der Ventilhülse 20 erfolgen. Die Verdrehung des Ventilkörpers 30 erfolgt um eine Drehachse, die auch die Längserstreckung des Stellventils 10 definiert, bei einer Verschiebung des Ventilkörpers 30 innerhalb der Ventilhülse 20 ist die Verschieberichtung die Längserstreckung des Stellventil 10.

Um eine Leckage zu vermeiden, sind an dem Außenumfang der Ventilhülse 20 zwei Dichtringe 25 angeordnet, die beiderseits des Strömungskanals 20 eine Abdichtung gegenüber einem Gehäuse oder der Hydraulikleitung bewirken. Zur Verdrehung oder Verlagerung des Ventilkörpers 30 relativ zu der Ventilhülse 20 ist an dem Ventilkörper 30 eine Verstelleinrichtung 50 angeordnet, über die der Ventilkörper verdreht werden kann. Die Verstelleinrichtung 50 ist in dem dargestellten Ausführungsbeispiel als eine Welle ausgebildet, die mit einem Antrieb oder einem manuell betätigbaren Stellelement oder Stellrad gekoppelt ist. Die Verstellung kann manuell oder über einen motorischen Antrieb erfolgen, der vorzugsweise über eine Steuerungseinrichtung sensorbasiert gesteuert wird, um eine Anpassung des hydraulischen Widerstandes auf der Grundlage von Sensordaten zu bewirken.

Innerhalb des Ventilkörpers 30 ist ein Hohlraum 31 ausgebildet, der in dem dargestellten Ausführungsbeispiel zylindrisch ausgebildet ist. Innerhalb des Hohlraums 31 ist ein Diffusoreinsatz 40 angeordnet, der drehfest und in Längserstreckung des Ventilkörpers 30 unverlagerbar innerhalb des Hohlraums 31 angeordnet ist. Das dem Auslass 3 gegenüberliegende Ende des Ventilkörpers 30 ist abgedichtet, insbesondere durch den Diffusoreinsatz 40 abgedichtet. Dazu weist der Diffusoreinsatz 40 ein im Wesentlichen zylindrisches Endstück auf, das innerhalb des Hohlraums 31 eingepasst ist. Das zylindrische Endstück reicht bis nahezu an die Durchlassöffnungen 34 heran, die in dem dargestellten Ausführungsbeispiel auf dem gleichen Umfangsbereich liegen, der ebenfalls wie die Hülsenwandöffnungen 24 zwischen den Dichtringen befindlich ist. Der Diffusoreinsatz 40 ist in dem dargestellten Ausführungsbeispiel als separates Bauteil ausgebildet, das innerhalb des Ventilkörpers 30 eingesetzt und festgelegt ist. Alternativ zu einer mehrteiligen Ausgestaltung kann der Diffusoreinsatz 40 auch einstückig mit dem Ventilkörper 30 ausgebildet sein, entweder durch ein additives Fertigungsverfahren oder durch ein entsprechendes Trennverfahren aus einem Vollmaterial hergestellt.

Der Diffusoreinsatz 40 verjüngt sich von seinem hinteren Ende, das den Hohlraum 31 verschließt, in Richtung auf den Auslass 30 und weist eine abgerundete Form auf, sodass radial von außen durch die Hülsenwandöffnung 24 und die Durchlassöffnung 34 strömendes Hydraulikfluid durch den Diffusoreinsatz um 90° in Richtung auf eine Zugangsöffnung 37, vorliegend eine Auslassöffnung 37 des Hohlraums 31 umgeleitet wird. Durch die sich in Richtung auf die Auslassöffnung 37 ergebende Veränderung der Form der Oberfläche des Diffusoreinsatzes 31 wird der Strömungsquerschnitt des Bereiches des Hohlraumes 31, der sich zwischen der Innenwand des Ventilkörpers 30 und dem Diffusoreinsatz 40 ausbildet, vergrößert. Durch die gerundete Oberflächenform mit der Krümmung in Richtung auf die Auslassöffnung 37 sowie die allmähliche Vergrößerung des Strömungsquerschnittes findet eine Umlenkung der Strömungsrichtung und eine Verlangsamung der Strömungsgeschwindigkeit statt, sodass Turbulenzen innerhalb des Hydraulikfluides vermieden oder zumindest vermindert werden.

Das Stellventil 10 weist mehrere Durchlassöffnungen 34 und Hülsenwandöffnungen 24 auf, die radial um die Ventilhülse 20 und den Ventilkörper 30 herum, vorzugsweise gleichbeabstandet, angeordnet sind. Dadurch ist es möglich, insgesamt einen vergleichsweise hohen Durchlass des Hydraulikfluides durch eine Vielzahl von Durchlassöffnungen 34 in den Hohlraum 31 eintreten zu lassen. Bei einer solchen Ausgestaltung mit mehreren Hülsenwandöffnungen 24 und Durchlassöffnungen 34 ist es vorteilhaft, in dem Bereich der Durchlassöffnungen 34 Trennwände 44, 45, 46 anzuordnen, die in der Figur 4 in der perspektivischen Darstellung des Diffusoreinsatzes 40 dargestellt sind. Der Diffusoreinsatz 40 in der Figur 4 ist ein einstückig ausgebildetes Formteil, das drei Trennwände 44, 45, 46 aufweist, die in dem eingebauten Zustand innerhalb des Ventilkörpers 30 bis zu der Innenwand des Hohlraumes 31 reichen. Bei einer zylindrischen Ausgestaltung des Hohlraumes 31 erstrecken sich die Trennwände 44, 45, 46 geradlinig von dem hinteren Ende des Diffusoreinsatzes 40 in Richtung auf den Auslass 3 bzw. die als Auslassöffnung ausgebildete Zugangsöffnung 37. Zwischen den hinteren Wänden 44, 45, 46 sind jeweils muldenartige, gerundete Strömungsflächen ausgebildet, die sich von dem Außenumfang des Ventilkörpers 40 radial nach innen in Richtung auf einen Zentralkörper 47 erstrecken. Der Zentralkörper 47 ist konisch ausgebildet und verjüngt sich in Richtung auf die Auslassöffnung 37. Der Zentralkörper 47 endet vorteilhafterweise vor der Auslassöffnung 37, um den gesamten Querschnitt des Hohlraumes 31 im Bereich der Auslassöffnung 37 als Strömungsquerschnitt für das Hydraulikfluid bereitzustellen. Die Trennwände 44, 45, 46 enden vor der Spitze des Zentralkörpers 47, wobei sich ein abgerundeter Übergang sowohl zwischen den Mulden zwischen den Trennwänden 44, 45, 46 als auch radial nach innen ergibt, um Turbulenzen innerhalb des Hydraulikfluides zu vermeiden.

An dem hinteren Ende des Diffusoreinsatzes 40 ist eine Ausnehmung als Verdrehsicherung ausgebildet, die formschlüssig in einen Vorsprung innerhalb des Ventilkörpers 30 eingreifen kann.

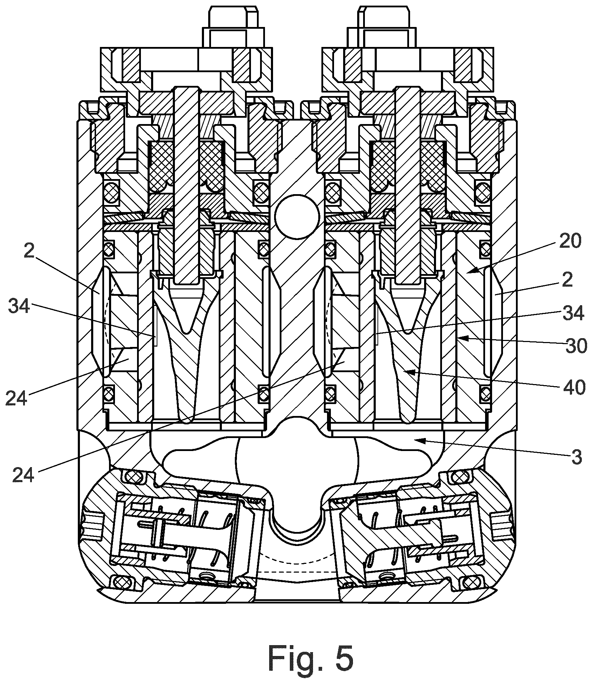

In der Figur 5 ist eine Querschnittsdarstellung zweier Stellventile 10 dargestellt, die parallel zueinander angeordnet sind und in einen gemeinsamen Auslass 3 münden. Von dem Auslass 3 kann das Hydraulikfluid durch Rückschlagventile zu der jeweiligen Kammer des hydraulischen Aktuators transportiert werden. Grundsätzlich ist es auch möglich, ohne Rückschlagventile eine umgekehrte Bewegung des Hydraulikfluides zuzulassen.

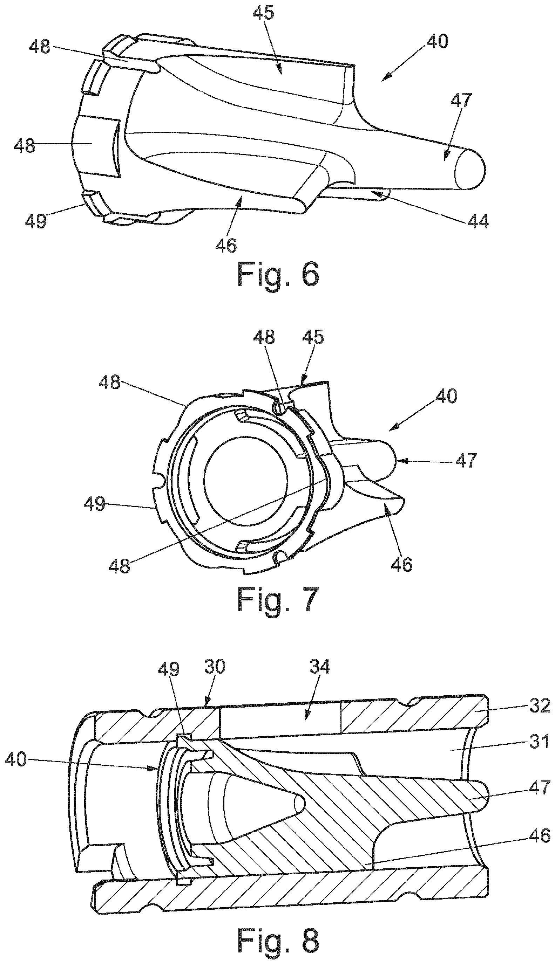

In der Figur 6 ist eine Variante des Diffusoreinsatzes 40 dargestellt. Auch dieser Diffusoreinsatz 40 weist drei Trennwände 44, 45, 46 auf, die über den Umfang gleichverteilt zueinander orientiert sind, sodass die Mittelebenen der Trennwände 44, 45, 46 in einem Winkel von 120° zueinander orientiert sind. An dem rückwärtigen Ende des Diffusoreinsatzes 40 sind Verdrehsicherungen 48 ebenso wie Axialsicherungen 49 ausgebildet, die eine Verschiebung und Verdrehung des Diffusoreinsatzes 40 innerhalb des Ventilkörpers 30 verhindern.

In der Figur 7 ist der Diffusoreinsatz 40 gemäß Figur 6 in einer rückwärtigen Ansicht dargestellt. Die Verdrehsicherungen 48 sowie die Axialsicherungen 49 an dem rückwärtigen Ende sind ebenso zu erkennen wie die Ausgestaltung als Hohlkörper und die Strömungsoberfläche, die sich in Richtung auf die Spitze des Zentralkörpers 47 in einer Krümmung nach vorne erstreckt, sodass sich der Diffusoreinsatz 40 in Strömungsrichtung von dem Einlass 2 zum Auslass 3 verjüngt. Auch die Trennwände 44, 45, 46 verjüngen sich in Richtung auf die Spitze des Zentralkörpers 47, vorzugsweise mit einer gekrümmten Kontur.

Figur 8 zeigt eine Querschnittsansicht des Ventilkörpers 30 mit dem eingesetzten Diffusoreinsatz 40 gemäß der Figuren 6 und 7. Der Außenumfang der Trennwände liegt dabei an der Innenwand des Ventilkörpers 30 an, dargestellt ist das Anliegen der Trennwand 46 an der Innenwand des Hohlraumes 31. Zwischen den beiden anderen Trennwänden ist die Durchlassöffnung 34 innerhalb der Ventilkörperwand 32 angeordnet, sodass drei voneinander getrennte Einströmbereiche innerhalb des Hohlraumes 31 durch die Trennwände 44, 45, 46 ausgebildet werden. Der radial von außen durch die Durchlassöffnungen 34 einströmende Hydraulikfluidstrom wird an der Oberfläche des Diffusoreinsatzes 40 um 90° umgelenkt und in Richtung auf die Auslassöffnung entlang des sich verjüngenden Zentralkörpers 47 entlang geleitet.

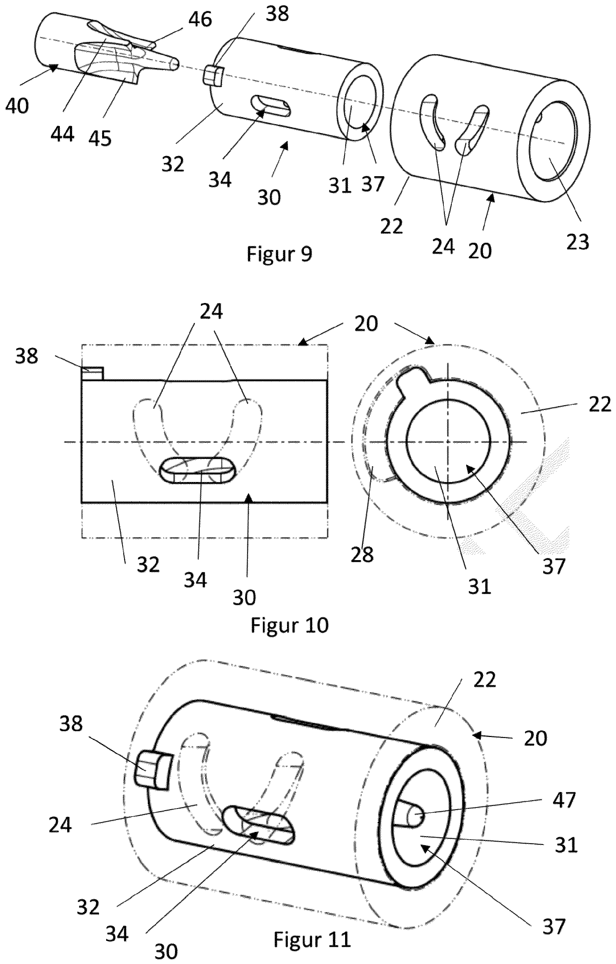

In der Figur 9 ist in einer Explosionsdarstellung das Stellventil mit den drei Hauptkomponenten Ventilhülse 20, Ventilkörper 30 und Diffusoreinsatz 40 dargestellt. Die Ventilhülse 20 ist zylindrisch ausgebildet und weist eine im Wesentlichen geschlossene Hülsenwand 22 auf, in der Hülsenwandöffnungen 24 um 120° versetzt um den Umfang herum gleichmäßig angeordnet sind. In dem dargestellten Ausführungsbeispiel sind die Hülsenwandöffnungen 24 durch zwei Teilöffnungen ausgebildet, die als gekrümmte Schlitze ausgebildet sind. Die Schlitze sind geneigt zur Längserstreckung der Ventilhülse 20 orientiert, alternativ können diese auch geradlinig und schräg zur Längserstreckung orientiert ausgebildet sein. Innerhalb der Ventilhülse 20 ist der Aufnahmeraum 23 als Zylinderschale und Aufnahmeraum für den Ventilkörper 30 ausgebildet. Die Hülsenwandöffnungen 24 durchdringen die Hülsenwand 22, sodass Fluid durch die Hülsenwandöffnung 24 in Richtung auf den Aufnahmeraum 23 und zu dem Ventilkörper 30 durch die Hülsenwand 22 hindurch treten kann.

Der Ventilkörper 30 weist in der dargestellten Ausführungsform ebenfalls einen zylindrischen Außenumfang auf. Der Ventilkörper 30 wird durch die Ventilkörperwand 32 als ein hülsenartiger Einsatz ausgebildet, bei dem in der Hülsenwand 32 drei Durchlassöffnungen über den Umfang um 120° versetzt in Umfangsrichtung hintereinander angeordnet sind. Die Durchlassöffnungen, von denen nur eine Durchlassöffnung 34 zu erkennen ist, sind in dem dargestellten Ausführungsbeispiel als ein geradliniger Schlitz ausgebildet, der sich entlang der Längserstreckung des Ventilkörpers 30 erstreckt. Der Ventilkörper 30 bildet den Hohlraum 31 mit der Zugangsöffnung 37 aus. An der Außenseite des Ventilkörpers 30 ist an dem rückwärtigen Ende, das der Zugangsöffnung 37 gegenüberliegt, ein Formschlusselement 38 als Anschlagbegrenzung angeordnet, um eine Verdrehung innerhalb der Ventilhülse 20 zu begrenzen.

Der Diffusoreinsatz 40 ist, analog zu den vorher beschriebenen Diffusoreinsätzen, mit drei Trennwänden 44, 45, 46 ausgebildet und wird drehfest und axial unbeweglich innerhalb des Hohlraumes 31 des Ventilkörpers 30 angeordnet. Alle Komponenten des Stellventils 10 sind koaxial orientiert und ineinander angeordnet. Alternativ zu einer Anordnung der Durchlassöffnung 34 als geradliniger Schlitz entlang der Längserstreckung des Ventilkörpers 30 kann diese auch geneigt zur Längserstreckung an dem Umfang der Ventilkörperwand 33 ausgebildet sein. Die Ausgestaltung kann ähnlich der Durchlassöffnungen 34 an der Hülsenwand 22 der Ventilhülse 20 ausgebildet sein. Ebenso kann die Hülsenwandöffnung 24 als längsorientierter Schlitz ausgebildet sein. Ebenso ist es möglich, dass beide Öffnungen, sowohl die Hülsenwandöffnung 24 als auch die Durchlassöffnung 34 wie die in der Figur 9 dargestellte Hülsenwandöffnung 24 ausgebildet sind, wobei die Abmessungen, Formgebungen und Orientierungen so ausgebildet sein müssen, dass zum vollständigen Schließen des Stellventils keinerlei Überlappungen der Öffnungen vorhanden sind. Zum Bewegen in die Offenstellung wird der Ventilkörper 30 relativ zu der Ventilhülse 20 verdreht, bis eine maximale Überdeckung der Öffnungen vorhanden ist.

In der Figur 10 sowie in der Figur 11 ist das Stellventil in der geöffneten Stellung dargestellt. Die Figur 10 zeigt einen Längsschnitt und einen Querschnitt, wobei die Ventilhülse 20 mit durchbrochenen Linien dargestellt ist. Figur 11 zeigt eine perspektivische Ansicht mit einer maximalen Durchtrittsöffnung, die durch die Überdeckung der Hülsenwandöffnung 24 über die gesamte Breite mit der Durchlassöffnung 34 erzielt wird. Innerhalb der Ventilhülse 20 ist ein Führungskanal 28 ausgebildet, in dem das Formschlusselement 38 des Ventilkörpers 30 geführt ist. In der Figur 10 in der rechten Darstellung ist die maximale Offenstellung mit dem Anschlag des Formschlusselementes 38 an dem Ende der Führung 28 dargestellt. In der Figur 11 ist zu erkennen, dass das vordere Ende des Zentralkörpers 47 kurz vor dem Ende der Zugangsöffnung 37 des Hohlraumes 31 des Ventilkörpers 30 endet. Das radial von außen einströmende Hydraulikfluid wird durch die Überdeckung der Hülsenwandöffnung 24 mit der Durchlassöffnung 34 hindurch in Richtung auf den Diffusoreinsatz 40 umgeleitet, wo das Hydraulikfluid auf die gekrümmte Oberfläche zwischen den Trennwänden 44, 45 trifft und dann in Richtung auf den Auslass 3 umgeleitet wird. Ein direktes Aufeinandertreffen der durch die Durchlassöffnungen 34 eintretenden Hydraulikfluidteilströme wird durch die vollständige hydraulische Trennung durch die Trennwände 44, 45, 46 erreicht, wobei sich die Trennwände 44, 45, 46 vorzugsweise bis zu dem Ende der Durchlassöffnungen 34 erstrecken.

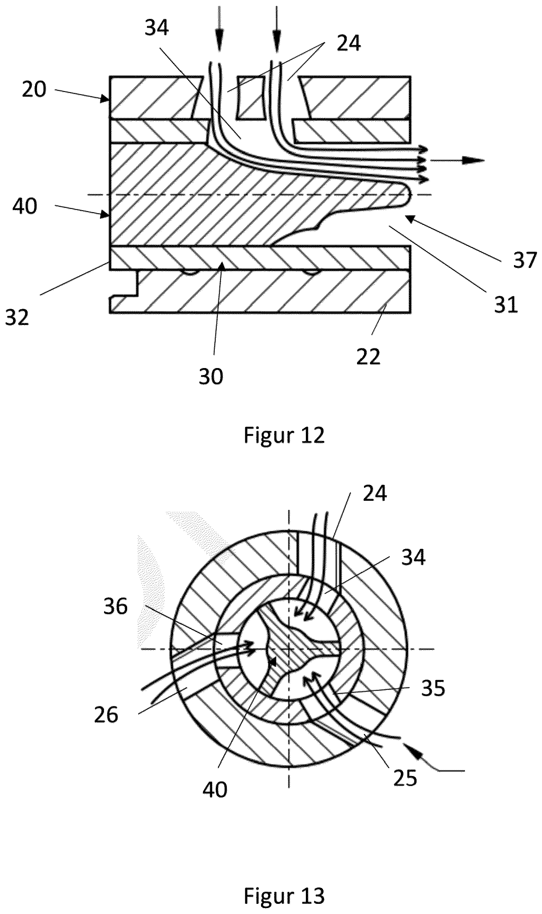

In der Figur 12 ist eine Längsschnittansicht des Stellventils 10 mit dem vorhandenen Strömungsweg dargestellt. Radial von außen tritt das Hydraulikfluid durch die Hülsenwandöffnungen 24 und die Durchlassöffnungen 34 in den Hohlraum 31 ein und wird an der Oberfläche des Diffusoreinsatzes 40 umgelenkt und in Richtung auf den Auslass 3 um 90° umgeleitet.

In der Figur 13 ist eine Querschnittsansicht des Stellventils 10 gemäß Figur 9 in montiertem Zustand gezeigt. Über den Umfang gleichmäßig verteilt sind die drei Hülsenwandöffnungen 24, 25, 26, die wiederum in strömungstechnischer Verbindung mit dem Strömungskanal bzw. dem Einlass stehen. Hydraulikfluid strömt durch die Hülsenwandöffnungen 24, 25, 26 und die korrespondierend dazu ausgerichteten Durchlassöffnungen 34, 35, 36 des Ventilkörpers 30 in den Hohlraum 31. Die Teilströme des Hydraulikfluides treffen auf den Ventilkörper 40 und werden in dem Bereich des Einströmens durch die Trennwände davon abgehalten, direkt aufeinander zutreffen. Die Kanäle, die durch die Trennwände ausgebildet werden, vereinigen sich in Richtung auf den Auslass 3, sodass ein gemeinsamer Fluidstrom des Hydraulikfluides an der Zugangsöffnung 37 anliegt.

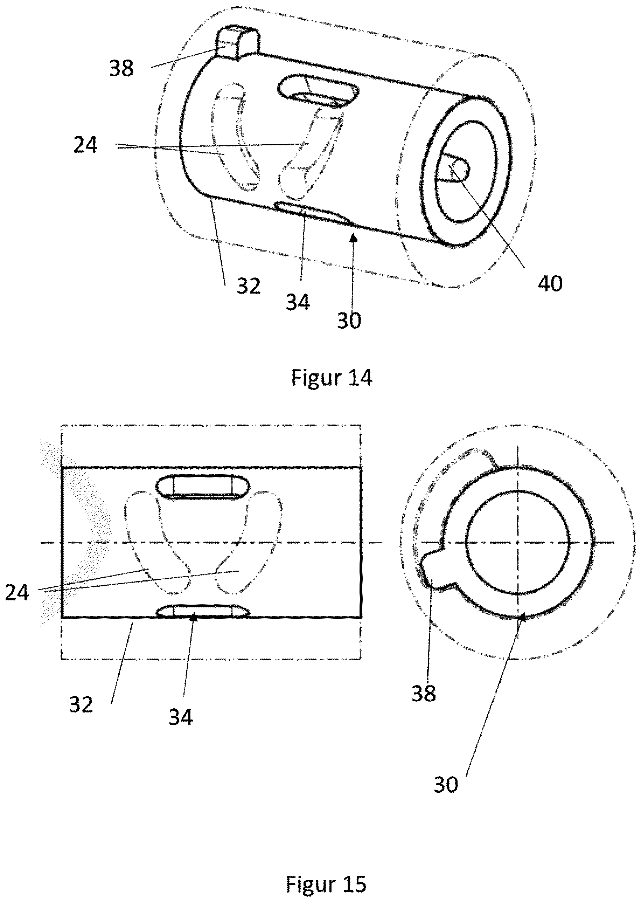

In den Figuren 14 und 15 ist das Stellventil in einer geschlossenen Stellung gezeigt. Der Aufbau entspricht im Wesentlichen dem der Figuren 11 und 10. In der Figur 15 ist zu erkennen, dass das Formschlusselement 38 innerhalb der Führung in der Ventilhülse bis zum gegenüberliegenden Anschlag verdreht ist. Dadurch ist der Ventilkörper 30 aus der Offenstellung in die dargestellte Schließstellung gebracht, in der keinerlei Überdeckung der Hülsenwandöffnung 24 mit der Durchlassöffnungen 34 vorhanden ist. Hydraulikfluid kann nicht von außen durch die Hülsenwandöffnung 24 und durch die Durchlassöffnungen 34 hindurchströmen, abgesehen von einer eventuell vorhandenen Leckage, die durch eine Minimierung der Spaltweiten minimiert oder ausgeschlossen werden kann.

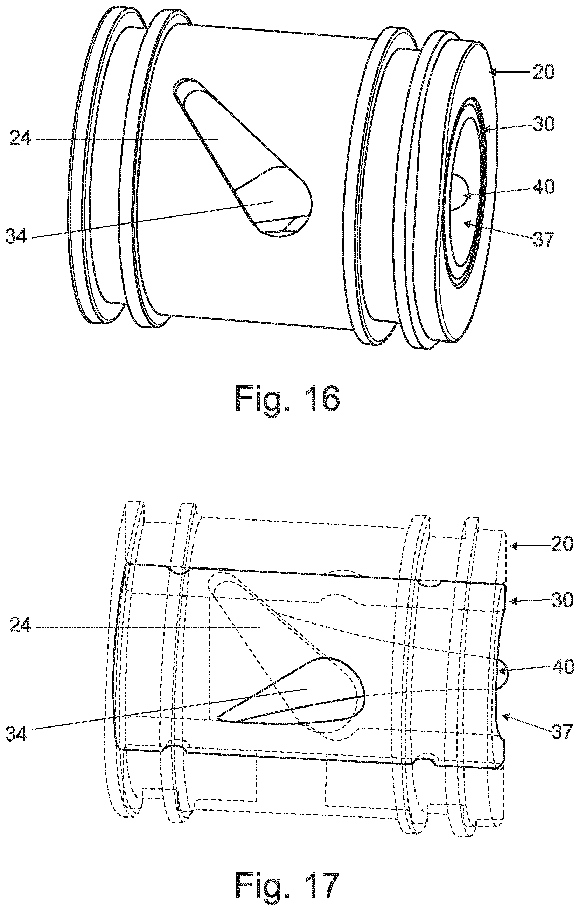

In der Figur 16 ist eine Variante des Stellventils 10 dargestellt, auch hier ist eine Ventilhülse 20 mit einem drehbar darin angeordneten Ventilkörper 30 und einem drehfest darin angeordneten und axial befestigten Diffusoreinsatz 40 dargestellt. Die Unterschiede zu den vorhergehenden Ausführungsformen beziehen sich auf die Geometrie und Ausgestaltung der Hülsenwandöffnungen 24 und der Durchlassöffnungen 34. Auch hier sind drei Hülsenwandöffnungen und drei Durchlassöffnungen vorgesehen, Alternativausgestaltungen mit nur zwei Hülsenwandöffnungen und Durchlassöffnungen bzw. mehr als drei Hülsenwandöffnungen und Durchlassöffnungen sowie gegebenenfalls mit nur einer Hülsenwandöffnung und einer Durchlassöffnung sind ebenfalls möglich.

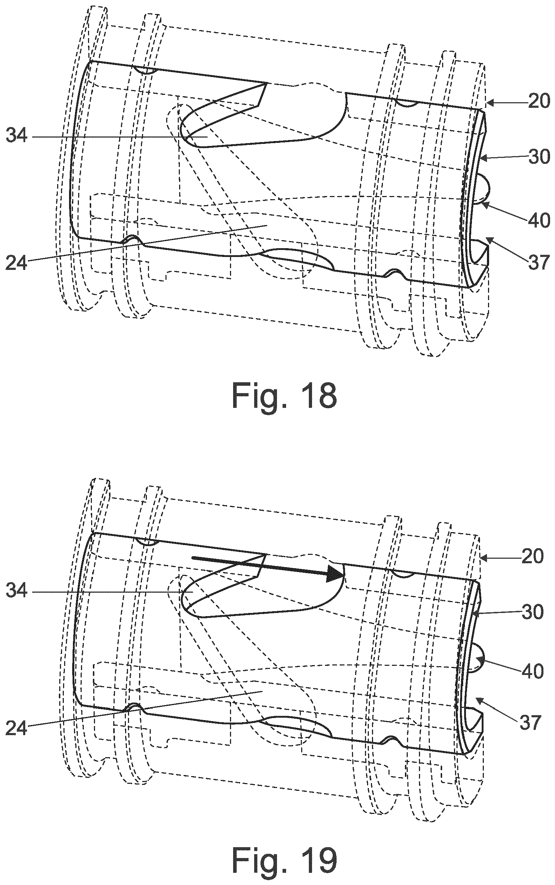

In dem dargestellten Ausführungsbeispiel sind die Hülsenwandöffnungen 24 als Schlitze ausgebildet, die geneigt zur Längserstreckung des Stellventils innerhalb der Wand des Ventilkörpers 30 sowie der Ventilhülse 20 ausgebildet sind. Die Neigung der Hülsenwandöffnungen 24 und die Neigung der Durchlassöffnungen 34 sind gegenläufig, sodass sich im Verlauf einer Drehung aus der dargestellten Offenstellung zu der in der Figur 18 und 19 dargestellten Schließstellung die Schlitze kreuzen. In der dargestellten Ausführungsform vergrößert sich die Breite der Schlitze von dem rückwärtigen Ende zu dem der Zugangsöffnung 37 zugewandten vorderen Ende kontinuierlich, da die Seitenflanken der Schlitze nicht parallel zueinander orientiert sind, sondern von hinten nach vorn in Richtung auf den Auslass auseinanderlaufen.

In den Figuren 18 und 19 befinden sich die Ventilhülse 20 und der Ventileinsatz 30 in einer nahezu geschlossenen Stellung. Der freie Durchtrittsquerschnitt, der sich aus der Überdeckung der Hülsenwandöffnungen 24 und der Durchlassöffnungen 34 ergibt, ist im Vergleich zu der Erstellung gemäß Figur 16 verkleinert. Die Position, an der das Hydraulikfluid sie in den Hohlraum 31 des Ventilkörpers 30 eintritt, wandert von ungefähr der Mitte des Ventilkörpers 30 an das hintere Ende in Richtung auf den Absatz des Diffusoreinsatzes, der an der Innenseite des Hohlraumes 31 des Ventilkörpers 30 anliegt. Der Pfeil in der Figur 19 deutet die Bewegungsrichtung des freien Durchtrittsquerschnittes in den Hohlraum 31 an, wenn der Ventilkörper 30 nach unten gedreht wird. Der freie Durchtrittsquerschnitt wandert dann von der hinteren Position nach unten und vorne. Aufgrund dieser Verlagerung der Position des freien Durchtrittsquerschnittes nach vorne wird der Weg des Hydraulikfluides von der Durchlassöffnungen zu der Oberfläche des Diffusoreinsatzes 40 größer, sodass sich eine optimierte Umleitung ergibt. Ein verringerter Volumenstrom aufgrund des kleineren Durchtrittsquerschnittes, wie er in den Figuren 18 und 19 dargestellt ist, kann näher an der Oberfläche des Diffusoreinsatzes 40 in den Hohlraum eintreten und in dem Kanal zwischen den Trennwänden hindurch in Richtung auf den Auslass geleitet werden.

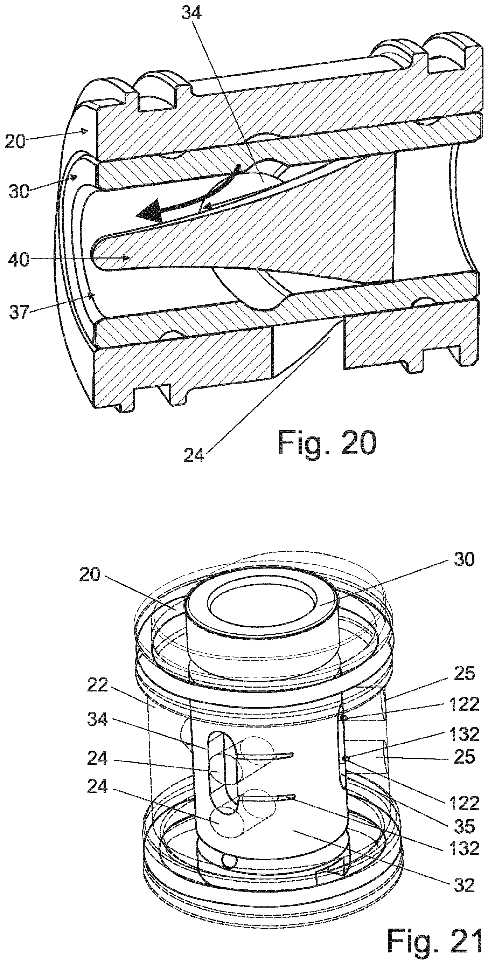

In der Figur 20 ist die Ausführungsform der Figuren 16 bis 19 in einer Querschnittsdarstellung gezeigt. Die Hülsenwandöffnung in der Ventilhülse 20 ist nicht gezeigt, die Durchlassöffnung 34 wandert relativ zu der Hülsenwandöffnung bei einer entsprechenden Drehung, wobei der freie Durchtrittsquerschnitt der sich überlagernden Hülsenwandöffnungen und Durchlassöffnungen sowohl in Umfangsrichtung als auch in Axialerstreckung wandert. In der Stellung gemäß der Figuren 18 und 19 tritt das Hydraulikfluid weiter am hinteren Ende in den Hohlraum 31 ein, was durch den längeren, hinteren Pfeil angedeutet ist. In der Offenstellung gemäß der Figuren 16 und 17 tritt ein größerer Volumenstrom durch den freien Durchtrittsquerschnitt der Hülsenwandöffnungen und Durchlassöffnungen hindurch, was durch den vorderen, dickeren Pfeil angedeutet ist.

Durch die Verstellung oder Verdrehung der Überdeckung von Hülsenwandöffnung und Durchlassöffnung wandert der freie Durchtrittsquerschnittes und damit die Einströmung des Hydraulikfluides in den Hohlraum in Richtung auf den Diffusoreinsatz 40 entlang der Längserstreckung. In Abhängigkeit von der Relativposition von Ventilhülse 20 und Ventilkörper 30 verändern sich die axiale Position des freien Durchtrittsquerschnittes und die Position des einströmenden Hydraulikfluides. Der Diffusoreinsatz 40 ist dabei so geformt, dass ein strömungstechnisches Optimum erreicht wird, sodass keine oder nur eine geringe turbulente Strömung beim Einströmen in das Stellventil vorhanden ist.

In der Figur 21 ist in einer teiltransparenten Ansicht das Stellventil mit der Ventilhülse 20 und dem darin angeordneten Ventilkörper 30 dargestellt. Auch hier ist der Ventilkörper 30 im Wesentlichen zylindrisch ausgebildet und drehbar in der Ventilhülse 20 gelagert. In der Hülsenwand 22 sind in der Längserstreckung oder Axialerstreckung voneinander beabstandet zwei Hülsenwandöffnungen 24 untereinander angeordnet. Insgesamt sind drei Paare von untereinander angeordneten Hülsenwandöffnungen 24 in Gestalt von Bohrungen um 120° verteilt herum um den Umfang der Ventilhülse 20 angeordnet, das zweite Paar Hülsenwandöffnungen 25 ist noch auf der rechten Seite zu erkennen. Korrespondierend zu der Ausgestaltung der Stellventile der vorigen Figuren sind Durchlassöffnungen 34, 35 ebenfalls um 120° um den Umfang verteilt an der Ventilkörperwand 32 ausgebildet, in dem dargestellten Ausführungsbeispiel als gerader Schlitz. Das Hydraulikfluid strömt in einer Ausgestaltung von den Hülsenwandöffnungen 24, 25 radial nach innen durch die Durchlassöffnungen 34, 35 in den Hohlraum innerhalb des Ventilkörpers 30 und von dort in den angeschlossenen Strömungskanal. Gegebenenfalls ist in dem Hohlraum ein Diffusor angeordnet. Unabhängig von der Anordnung eines Diffusors in dem Hohlraum sind an der Außenseite der Ventilkörperwand 32 Kanäle 132 eingearbeitet, die einen halbrunden Querschnitt aufweisen und sich in Umfangsrichtung parallel zueinander von der Durchlassöffnung 34, 35 weg erstrecken. Die Kanäle 132 sind als nach außen offene Kanäle ausgebildet, die durch die Hülsenwand 22 geschlossen werden. Die Länge in Umfangsrichtung ist so bemessen, dass bei einer Verdrehung des Ventilkörpers 30 relativ zu der Ventilhülse 20 die schlitzartige Durchlassöffnung 34 soweit verdreht werden kann, dass die Hülsenwandöffnungen 24 nicht mehr in strömungstechnischer Verbindung mit den Kanälen einer 32 stehen, allerdings auch nicht mit der nachfolgenden Durchlassöffnung 35 in Kontakt treten. Der Abstand zwischen dem Kanal 132 und der nicht damit verbundenen Durchlassöffnung ist so groß, dass die Hülsenwandöffnung 24 einer glatten Ventilkörperwand 32 gegenüberliegt. Ebenfalls sind in der Ausführungsform der Figur 21 in der Ventilhülse 20 Rinnen oder Kanäle 122 eingearbeitet, die in der Hülsenwand 22 nach innen offen sind und korrespondierend zu den Kanälen 132 des Ventilkörpers 30 ausgebildet sind. Bevorzugt weisen die Rinnen oder Kanäle 122, 132 eine im Wesentlichen konstante Breite und/oder Tiefe auf, so dass sich in dem ggf. zusammengesetzten Kanal eine laminare Strömung ausbildet.

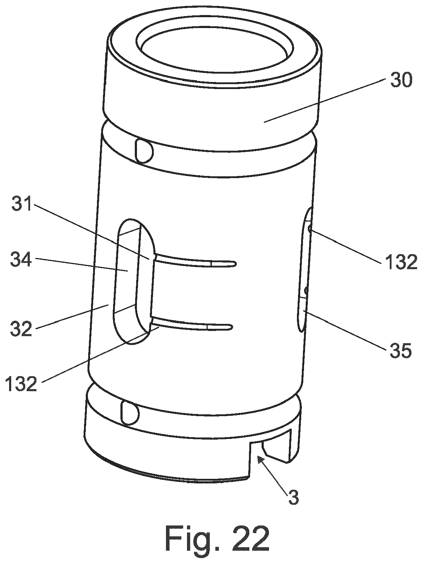

In der Figur 22 ist der Ventilkörper 30 des Stellventils gemäß Figur 21 in Einzeldarstellung gezeigt. Der Ventilkörper 30 ist als Buchse oder Hülse ausgebildet und weist eine Ventilkörperwand 32 auf, deren Außenseite Nuten für die Aufnahme von Dichtungsringen angeordnet sind. Ebenfalls sind Durchlassöffnungen 34, 35 in der Ventilkörperwand 32 eingearbeitet, die bis zu einem Hohlraum 31 innerhalb des Ventilkörpers 30 erreichen. Die Oberseite des Ventilkörpers 30 ist verschlossen, an der Unterseite mündet der Hohlraum 31 in einen Auslass 3, der wiederum zu einem Strömungskanal führt, wie oben beschrieben. Bei einer umgekehrten Strömungsrichtung, also bei einer Strömungsrichtung, bei der das Hydraulikfluid zuerst in den Hohlraum 31 eintritt, durch die Durchlassöffnungen 34, 35 hindurch zu den Hülsenwandöffnungen geleitet wird, ist die Zugangsöffnung ein Einlass.

In der Figur 22 ist zu erkennen, dass die Kanäle 132 sich nur über einen Teilumfang der Außenseite der Ventilkörperwand 32 erstrecken. Die Kanäle münden einseitig in die Durchlassöffnung 34, 35, wobei sich der Querschnitt bzw. die Tiefe des Kanals 132 in Richtung auf die Durchlassöffnung 34, 35 vergrößert. Die Vergrößerung erfolgt möglichst ohne Sprünge, um turbulente Strömungen zu vermeiden. Abweichend von der dargestellten geradlinigen Ausführung der Kanäle 132 um den Umfang der Außenseite der Ventilkörperwand 32 können diese auch eine Biegung in Axialerstreckung ausführen, sodass die Kanäle 132 nicht auf der gleichen Höhe des Ventilkörpers 32 verlaufen.

Durch die in Richtung auf die Durchlassöffnungen 34, 35 gerichteten Kanäle 132 ist es möglich, eine Drosselgeometrie bereitzustellen, die für eine laminare Drosselung sorgt. Dadurch werden Strömungsgeräusche verringert und starke lokale Aufheizungen an der Drosselstelle in dem Stellventil vermindert. Die Kanäle 132 können auch bei allen andere oben beschriebenen Formen und Konfigurationen von Durchlassöffnungen und Hülsenwandöffnungen angeordnet werden. Sind die Kanäle 132 in dem Ventilkörper 30 angeordnet, werden die Durchlassöffnungen 34, 35 durch diese Kanäle 132 ergänzt. Bei einem nicht vollständig geöffneten Stellventil und beispielsweise einer Zuführung des Hydraulikfluides durch die Ventilhülse 20 trifft das Hydraulikfluid zumindest teilweise auf die Kanäle 132, sodass das Hydraulikfluid zunehmend eine längere Strecke zurücklegen muss, um zu dem jeweiligen Ventilausgang zu gelangen. Die Charakteristik dieses Strömungsweges ist laminar. Alternativ oder ergänzend sind die Kanäle 122 oder Kanäle 122 an der Innenseite der Hülsenwand 22 ausgebildet oder angeordnet, was beispielsweise bei einer Umkehr der Strömungsrichtung für eine entsprechende Beruhigung der Strömung bzw. eine Vermeidung einer turbulente Strömung sorgt.

## Patentansprüche

1. Stellventil (10) für einen hydraulischen Aktuator, insbesondere für orthopädietechnische Einrichtungen, in einem Strömungskanal (1),
- das Stellventil (10) weist eine Ventilhülse (20) und einen Ventilkörper (30) auf,
- die Ventilhülse (20) weist eine Hülsenwand (22) auf, die einen Aufnahmeraum (23) ausbildet, in der Hülsenwand (22) ist zumindest eine radial ausgerichtete Hülsenwandöffnung (24, 25, 26) ausgebildet,
- der Ventilkörper (30) ist innerhalb des Aufnahmeraumes (23) der Ventilhülse (20) verlagerbar zwischen einer Schließstellung und einer Offenstellung angeordnet und weist einen durch den Ventilkörper (30) gebildeten Hohlraum (31) auf,
- der Ventilkörper (30) weist zumindest eine Ventilkörperwand (32) auf, die der zumindest einen Hülsenwandöffnung (24, 25, 26) gegenüberliegt und in der eine Durchlassöffnung (34, 35, 36) ausgebildet ist, die in der Offenstellung der korrespondierenden Hülsenwandöffnung (24, 25, 26) gegenüberliegt,
**dadurch gekennzeichnet, dass** in der Hülsenwand (22) und/oder der Ventilkörperwand (32) zumindest ein Kanal (122, 132) ausgebildet ist und der in der Hülsenwand (22) ausgebildete Kanal (122) an der Hülsenwandöffnung (24, 25, 26) und der an der Ventilkörperwand (32) ausgebildete Kanal (132) an der Durchlassöffnung (34, 35, 36) mündet.

2. Stellventil nach Anspruch 1, **dadurch gekennzeichnet, dass** ein an der Hülsenwand (22) ausgebildeter Kanal (122) einem an der Ventilkörperwand (32) ausgebildeten Kanal (132) gegenüberliegt.

3. Stellventil nach Anspruch 1 oder 2, **dadurch gekennzeichnet**, das der Kanal (122, 132) zumindest teilweise als nach außen offener Kanal oder Rinne ausgebildet ist.

4. Stellventil nach einem der voranstehenden Ansprüche, **dadurch gekennzeichnet, dass** der Kanal (122, 132) sich in Richtung auf die Hülsenwandöffnung (24, 25, 26) und/oder Durchlassöffnung (34, 35, 36) vergrößert.

5. Stellventil nach einem der voranstehenden Ansprüche, **dadurch gekennzeichnet, dass** der Hohlraum (31) bis auf die zumindest eine Durchlassöffnung (34, 35, 36) und eine Zugangsöffnung (37) geschlossen ausgebildet ist.

6. Stellventil nach einem der voranstehenden Ansprüche, **dadurch gekennzeichnet, dass** der Ventilkörper (30) drehbar oder verschieblich in der Ventilhülse (20) gelagert ist.

7. Stellventil nach einem der voranstehenden Ansprüche, **dadurch gekennzeichnet, dass** der Ventilkörper (30) mit einer Verstelleinrichtung (50) gekoppelt ist.

8. Stellventil nach einem der voranstehenden Ansprüche, **dadurch gekennzeichnet, dass** die Ventilhülse (20) als Teil eines Gehäuses (4) oder als separates Bauteil ausgebildet ist.

9. Stellventil nach einem der voranstehenden Ansprüche, **dadurch gekennzeichnet, dass** die Hülsenwandöffnung (24, 25, 26) und/oder die Durchlassöffnung (34, 35, 36) als Schlitz oder Bohrung ausgebildet sind/ist

10. Stellventil nach Anspruch 9, **dadurch gekennzeichnet, dass** der Schlitz schräg zur Längserstreckung des Stellventils (10) orientiert ist.

11. Stellventil nach Anspruch 9 oder 10, **dadurch gekennzeichnet, dass** der Schlitz geradlinig oder gekrümmt ausgebildet ist und/oder sich entlang seiner Längserstreckung in seiner Breite verändert.

12. Stellventil nach einem der voranstehenden Ansprüche, **dadurch gekennzeichnet, dass** die Kanäle (122, 132) so angeordnet oder ausgebildet sind, dass in der Schließstellung keine strömungstechnische Verbindung zwischen der Hülsenwandöffnung (24, 25, 26) und der Durchlassöffnung (34, 35, 36) besteht.

13. Stellventil nach einem der voranstehenden Ansprüche, **dadurch gekennzeichnet, dass** der Hohlraum (31) in einen Auslass (3) mündet und in dem Hohlraum (31) ein Diffusoreinsatz (40) angeordnet ist, der das Hydraulikfluid von der Durchlassöffnung (34 35, 36) in Richtung auf den Auslass (3) umlenkt.

14. Stellventil nach Anspruch 13, **dadurch gekennzeichnet, dass** der Diffusoreinsatz (40) separat von dem Ventilkörper (30) gefertigt und an oder in dem Ventilkörper (30) befestigt oder integraler Bestandteil des Ventilkörpers (30) ist.

15. Stellventil nach einem der Ansprüche 13 oder 14, **dadurch gekennzeichnet, dass** der Ventilkörper (30) mehrere Durchlassöffnungen (34, 35, 36) und der Diffusoreinsatz (40) Trennwände (44, 45, 46) aufweist, die zwischen den Durchlassöffnungen (34, 35, 36) angeordnet sind.

16. Stellventil nach Anspruch 15, **dadurch gekennzeichnet, dass** die Trennwände (44, 45, 46) sich bis zu dem Auslass (3) erstrecken oder davor enden.

17. Stellventil nach einem der Ansprüche 13 bis 16, **dadurch gekennzeichnet, dass** der Diffusoreinsatz (40) in Richtung auf den Auslass (3) sich verjüngend ausgebildet ist.

18. Stellventil nach Anspruch 3, **dadurch gekennzeichnet, dass** der Kanal (122, 132) derart ausgeführt ist, dass sich eine laminare Strömung in dem Kanal (122, 132) ausbildet und ein proportionaler Zusammenhang zwischen Druck und Volumenstrom vorliegt.

19. Stellventil nach Anspruch 3 oder 18, **dadurch gekennzeichnet, dass** der Kanal (122, 132) eine konstante Breite und/oder eine konstante Tiefe aufweist.
